# EUROPEAN PATENT SPECIFICATION

(11) **EP 3 705 099 B1**
(45) Date of publication and mention of the grant of the patent: **06.09.2023**
(21) Application number: 18896123.9
(22) Date of filing: 17.12.2018
(51) Int. Cl.: A61F 13/53, A61F 13/15, A61F 13/49, A61F 13/56

(54) **ABSORBENT ARTICLE**
ABSORBIERENDER ARTIKEL
ARTICLE ABSORBANT

(30) Priority: 28.12.2017 JP 2017254987
(43) Date of publication of application: 09.09.2020
(73) Proprietor: Unicharm Corporation, Shikokuchuo-shi, Ehime 799-0111 (JP)
(72) Inventor: SO, Tatsuya, Kanonji-shi, Kagawa 769-1602 (JP); KUDO, Etsuko, Kanonji-shi, Kagawa 769-1602 (JP); YAMASHITA, Junko, Kanonji-shi, Kagawa 769-1602 (JP); HASHINO, Yuki, Kanonji-shi, Kagawa 769-1602 (JP)
(74) Representative: Dolleymores
(86) International application number: PCT/JP2018/046292
(87) International publication number: WO 2019/131266

(56) References cited:
- EP-A1- 2 835 116
- EP-A1- 3 701 923
- WO-A1-2014/073635
- WO-A1-2015/046631
- WO-A1-2015/156067
- WO-A1-2015/159610
- WO-A1-2015/182307
- WO-A1-2017/169380
- WO-A1-2019/131265
- WO-A1-2019/131269
- JP-A- H06 296 644
- JP-A- 2002 165 833
- JP-A- 2006 043 067
- JP-A- 2006 271 898
- JP-A- 2010 012 002
- JP-A- 2010 162 338
- JP-A- 2012 157 380
- JP-A- 2016 030 206
- US-A1- 2005 148 988

## Description

### [TECHNICAL FIELD]

The present invention relates to an absorbent article such as a disposable diaper.

An absorbent article such as a disposable diaper described in Patent Literature 1 includes a front waistline region disposed around the front waistline of a wearer and a rear waistline region disposed around a rear waistline of the wearer, and a crotch region disposed in the crotch portion of the wearer. An absorbent core that absorbs body fluid of the wearer extends from the front waistline region to the rear waistline region.

In the absorbent article described in Patent Literature 1, the rear waistline region is provided with a sheet-like elastic element stretchable in the width direction. The absorbent core has a low rigidity region where a basis weight is lower than that in another portion of the absorbent core or no absorbent core is present. The low rigidity region is the center of the absorbent core in the width direction and extends forward from the rear end edge of the absorbent core. In the absorbent article described in Patent Literature 1, it is said that the low rigidity region of the absorbent core and the sheet-like elastic element increase the fitness to the wearer's buttocks.

### [CITATION LIST]

### [PATENT LITERATURE]

[Patent Literature 1] JP 2013-138702 A

WO 2015/182307 A1, EP 2835116 A1, WO 2014/073535 A1 and WO 2015/156067 A1 all disclose absorbent articles with absorbent cores having a low rigidity region where a basis weight is lower than that in another portion of the absorbent core or no absorbent core is present. The low rigidity region is in the center of the absorbent core in the width direction and extends forward from the rear end edge of the absorbent core. They also disclose notches formed in the absorbent core in the crotch region.

WO 2017/169380 A1 relates to disposable diaper in which an inner body that has an absorber can be moved vertically in accordance with a wearer's lateral decubitus position. In this respect, the inner body is formed with a liquid-permeable surface sheet, a non-liquid-permeable rear surface sheet, and the absorber provided between the surface sheet and the rear surface sheet; longitudinal front and back end portions of the inner body are fixed to an inner surface of an outer body, and the portion between the longitudinal front and back end portions of the inner body are not fixed to the inner surface of the outer body.

### [SUMMARY OF INVENTION]

With regard to absorbent articles such as disposable diapers, there is still room for improvement regarding the problems of fitness to the wearer's buttocks and suppression of leakage of body fluid. In particular, the inventor of the present invention has found an absorbent article that makes it easier to deform the portion of the absorbent core corresponding to the wearer's buttocks into a cup shape during wearing and can suppress leakage of body fluid.

An absorbent article according to one aspect includes a front-rear direction ,a width direction orthogonal to the front-rear direction, a front waistline region, a rear waistline region, a crotch region arranged between the front waistline region and the rear waistline region, an absorbent core extending at least from the crotch region to the rear waistline region, and an elastic member at least partially overlapping the absorbent core in the rear waistline region and expanding and contracting in the width direction. A folding line for folding the absorbent article in half extends along the width direction in the crotch region. The absorbent core has a pair of side slits located at both outside edges of the absorbent core in the width direction. An inner edge part located on an innermost side of the pair of side slits in the width direction is located on a rear side relative to a line which is in the middle between a rear end edge of the absorbent core and the folding line, wherein the absorbent core has a rear slit extending forward from a rear end edge in the rear waistline region.

### [BRIEF DESCRIPTION OF DRAWINGS]

Fig. 1 is a schematic view of an absorbent article according to an embodiment.
Fig. 2 is an exploded plan view of an absorbent article according to an embodiment.
Fig. 3 is a cross-sectional view of the absorbent article cut along line A-A of Fig. 2.
Fig. 4 is a plan view of an absorbent core of an absorbent article.
Fig. 5 is a schematic view of an absorbent core when receiving force on the inside of a rear region in a width direction.

### [DESCRIPTION OF EMBODIMENTS]

### (1) Outline of Embodiment

According to the present specification and the accompanying drawings, at least the following matters are disclosed.

The present invention provides the absorbent article of independent claim 1. The dependent claims specify preferred but optional features.

An absorbent article according to one aspect includes a front-rear direction ,a width direction orthogonal to the front-rear direction, a front waistline region, a rear waistline region, a crotch region arranged between the front waistline region and the rear waistline region, an absorbent core extending at least from the crotch region to the rear waistline region, and an elastic member at least partially overlapping the absorbent core in the rear waistline region and expanding and contracting in the width direction. A folding line for folding the absorbent article in half extends along the width direction in the crotch region. The absorbent core has a pair of side slits located at both outside edges of the absorbent core in the width direction. An inner edge part located on an innermost side of the pair of side slits in the width direction is located on a rear side relative to a line which is in the middle between a rear end edge of the absorbent core and the folding line. The absorbent core has a rear slit extending forward from a rear end edge in the rear waistline region.

By the action of the elastic member expanding and contracting in the width direction, the absorbent core receives a force inward in the width direction in the rear waistline region. Here, the pair of side slits serve as the folding origin of the absorbent core, and the absorbent core easily deforms inward in the width direction from the pair of side slits as the origin in the rear waistline region, and is easily bent in the longitudinal direction from an imaginary line connecting together the inner edge parts located at the innermost sides of the pair of side slits as the origin. Due to this, the outer end part in the width direction of the absorbent core in the rear waistline region moves inward in the width direction and deforms toward the skin side. By this deformation, the part of the absorbent core corresponding to the wearer's buttocks becomes easy to have a cup shape in response to the curvature of the wearer's buttocks.

Furthermore, since the pair of side slits serve as the folding origin of the absorbent core, the absorbent core on the rear side relative to the imaginary line connecting together the inner edge parts located on the innermost side of the pair of side slits can be suppressed from deforming with the deformation of the absorbent core in the crotch region. Accordingly, during wearing, the absorbent core on the rear side relative to the imaginary line connecting together the inner edge parts located on the innermost side of the pair of side slits becomes easy to maintain a state of being fitted to the body on the dorsal side of the wearer.

According to a preferred aspect, the absorbent core has a constricted region constricted inward in the width direction at least in the crotch region. The inner edge part located on an innermost side of the pair of side slits is located on a rear side relative to a position where a width of the absorbent core is narrowest in the constricted region.

The constricted region of the absorbent core is a region sandwiched by the legs of the wearer. Accordingly, in the constricted region, in particular the position where the width of the absorbent core is the narrowest, the rigidity becomes high by crush of the absorbent core in the width direction. Since the inner edge parts of the pair of side slits are provided at positions away from the region rearward, it is possible to further function the action of deforming the absorbent core with the side slits as the origin.

According to a preferred aspect, the inner edge part located on an innermost side of the pair of side slits is located on a rear side relative to the constricted region.

Since the inner edge parts of the pair of side slits are provided at positions away from the constricted region of the absorbent core rearward, it is possible to further function the action of deforming the absorbent core with the side slits as the origin.

According to a preferred aspect, a maximum length of the pair of side slits in the front-rear direction is longer than a thickness of the absorbent core.

The absorbent cores adjacent to each other in the front-rear direction with respect to the side slits can be suppressed from overlapping each other when the absorbent core deforms into a cup shape in the rear waistline region. This makes it easy for the absorbent core to have a rounded shape in accordance with the wearer's body.

According to a preferred aspect, the elastic member includes a rear-side elastic member on a rear side relative to the pair of side slits, and a front-side elastic member on a front side relative to the pair of side slits. A contraction force of the rear-side elastic member is higher than a contraction force of the front-side elastic member.

Due to this, the absorbent core located on the rear side relative to the pair of side slits receives a force directed inward in the width direction more strongly. Accordingly, the outside part in the width direction of the absorbent core in the rear waistline region deforms so as to move further inward in the width direction, and becomes easy to have a cup shape in response to the curvature of the wearer's buttocks.

According to a preferred aspect, a region between the folding line and the pair of side slits is not provided with an elastic member extending and contracting in the width direction.

Due to this, the region on the rear side relative to the pair of side slits receives a force directed inward in the width direction more strongly than the region on the front side relative to the pair of side slits does. Accordingly, the outside part in the width direction of the absorbent core in the rear waistline region deforms so as to move further inward in the width direction, and becomes easy to have a cup shape in response to the curvature of the wearer's buttocks.

According to a preferred aspect, the absorbent article includes a side engaging portion engaging the front waistline region and the rear waistline region. The pair of side slits are provided in a region overlapping the side engaging portion when viewed from the width direction.

The region overlapping the side engaging portion when viewed from the width direction is a region fitted to the waistline of the wearer. Therefore, the absorbent core is easy to receive a force inward in the width direction in the region overlapping the side engaging portion. Since the pair of side slits are provided in the region overlapping the side engaging portion when viewed from the width direction, the outside part in the width direction of the absorbent core in the rear waistline region becomes easier to move inward in the width direction, and becomes easy to have a cup shape in response to the curvature of the wearer's buttocks.

According to a preferred aspect, the absorbent article includes a fastening tape provided in the rear waistline region and fastening the rear waistline region to the front waistline region. The pair of side slits are provided on a front side relative to the fastening tape.

The fastening tape is fastened in a state of being pulled outward in the width direction. Since the front side (crotch side) relative to the fastening tape is provided with the pair of side slits, the contraction force is not inhibited from acting inward in the width direction at the position of the pair of side slits. For this reason, the outside part in the width direction of the absorbent core in the rear waistline region becomes easier to move inward in the width direction, and becomes easy to have a cup shape in response to the curvature of the wearer's buttocks.

According to the present invention, the absorbent core has a rear slit extending forward from a rear end edge in the rear waistline region.

The absorbent core in the rear waistline region tends to deform inward in the width direction from the side slit as the origin. Here, since the rear waistline region is provided with the rear slit extending forward from the rear side edge, the absorbent core becomes easier to deform inward in the width direction.

According to a preferred aspect, a part located on an innermost side of the side slit in the width direction is provided on a front side relative to a front side edge of the rear slit. In the case where the absorbent core deforms inward in the width direction in the rear waistline region, distortion due to the deformation of the absorbent core can occur up to the front side edge of the rear slit. Since the part located on the innermost side of the side slit in the width direction is provided on the front side relative to the front side edge of the rear slit, the side slit can mitigate this distortion. Accordingly, deformation of the absorbent core in the rear waistline region is difficult to be transmitted to the crotch region, and only the absorbent core in the rear waistline region is easy to deform into a desired shape.

According to a preferred aspect, a part located on an innermost side of the side slit in the width direction is located near a front side edge of the rear slit relative to the middle line between a rear end edge of the absorbent core and the folding line.

By bringing the part located on the innermost side of the side slit close to the front side edge of the rear slit, the absorbent core on the rear side relative to the side slit can be made easier to deform inward in the width direction by the action of both the side slit and the rear slit. This makes it easy for the absorbent core in the rear waistline region to have a cup shape corresponding to the curvature of the wearer's buttocks.

According to a preferred aspect, the rear slit is provided on both sides with respect to a center in the width direction.

The rear slits are provided on both sides with respect to the center in the width direction, and the absorbent core exists between the pair of rear slits. Therefore, even in the case where the body fluid runs immediately behind from the center in the width direction of the absorbent core, the absorbent core between the pair of rear slits can absorb the body fluid. Due to this, even if the rear slit making it easy to deform the absorbent core exists, it is possible to suppress the leakage of the body fluid from behind.

According to a preferred aspect, an outer contour outside in the width direction of the pair of rear slits has a part inclining rearward and outward in the width direction.

Since an outer contour of the rear slit obliquely inclines, the absorbent core is easier to deform so as to form a cup shape when the absorbent core in the rear waistline region receives a force inside in the width direction.

According to a preferred aspect, an outer contour inside in the width direction of the pair of rear slits has a part inclining rearward and outward in the width direction.

Since an outer contour of the rear slit obliquely inclines, the absorbent core is easier to deform so as to form a cup shape when the absorbent core in the rear waistline region receives a force inside in the width direction.

According to a preferred aspect, a front end edge of the rear slit is pointed inward in the width direction.

Due to this, the absorbent core on the outside relative to the rear slit is easy to deform inward around the front end edge of the rear slit. Accordingly, the absorbent core becomes easier to deform so as to form a cup shape in the rear waistline region.

### (2) Absorbent article according to the embodiment

The absorbent article according to the embodiment will be described below with reference to the drawings. It is to be noted that in the following drawings, identical or similar parts are denoted by the identical or similar reference numerals. However, it is to be noted that the drawings are schematic, and the proportions of each dimension are different from the actual ones. Accordingly, specific dimensions should be determined in consideration of the following explanation. It is also possible to include parts having different dimensional relationships and proportions among the drawings.

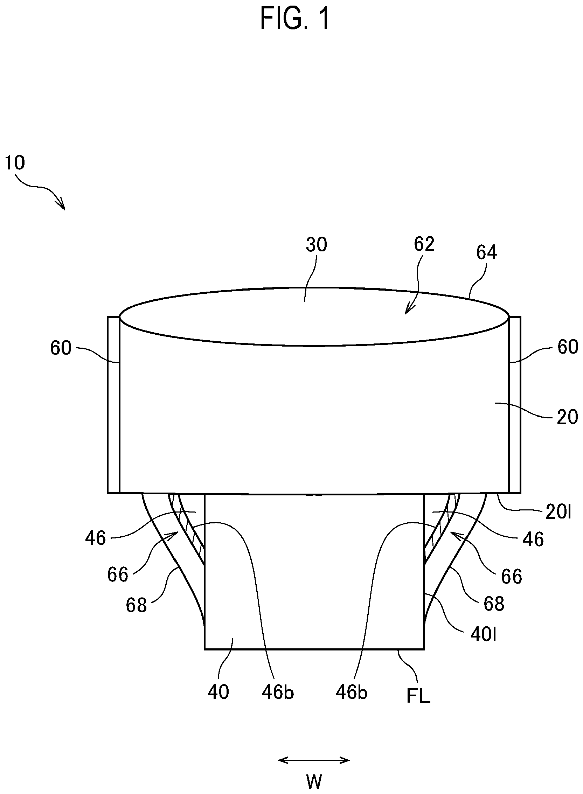

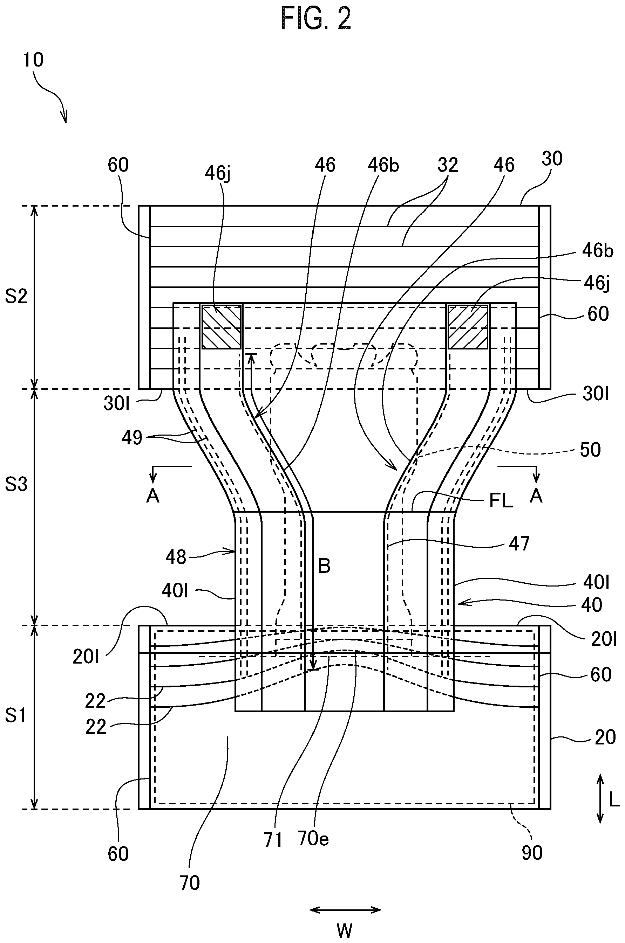

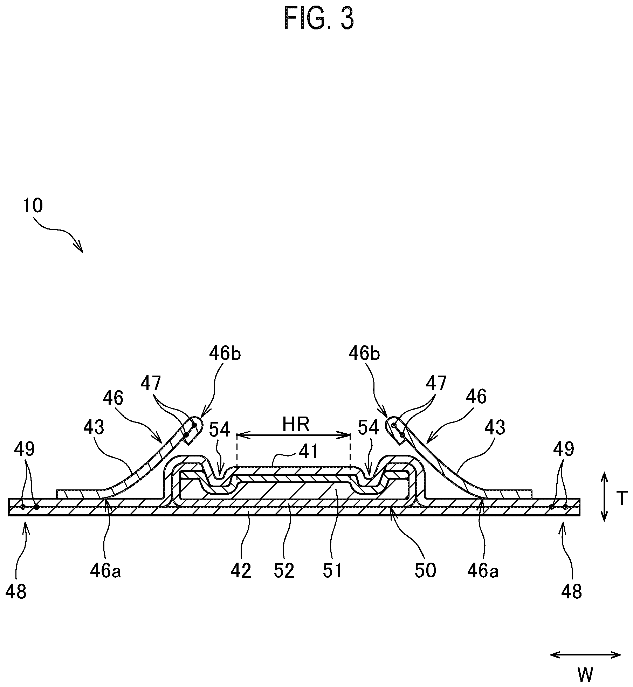

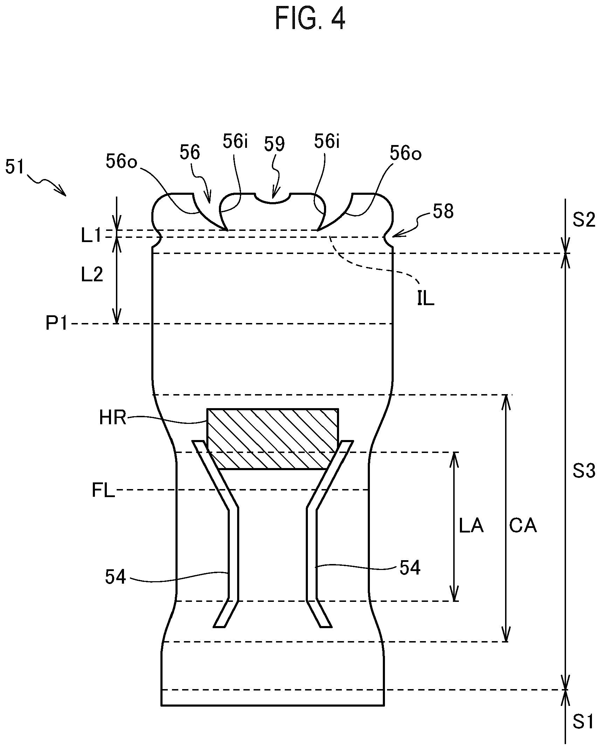

Fig. 1 is a schematic view of an absorbent article according to an embodiment. Fig. 2 is a developed plan view of an absorbent article according to an embodiment. Fig. 2 illustrates an extended state of the absorbent article extended till no wrinkles are formed in a state in which a side engaging portion described later is developed. Fig. 3 is a cross-sectional view of the absorbent article taken along line A-A of Fig. 2. Fig. 4 is a plan view of an absorbent core of an absorbent article.

An absorbent article 10 includes a front-back direction L and a width direction W, both directions being orthogonal to each other in a developed state. The front-back direction L is defined by a direction extending to the front (ventral) side and the rear (dorsal) side of the body. In other words, the front-back direction L is a direction extending forward and backward in the developed absorbent article 10. Further, the absorbent article 10 includes a thickness direction T perpendicular to both the front-back direction L and the width direction W

The absorbent article 10 includes a front waistline region S1, a rear waistline region S2, and a crotch region S3. The front waistline region S1 is a region facing the front waistline of the wearer. The rear waistline region S2 is a region facing the rear waistline of the wearer. The crotch region S3 is a region located in the crotch of the wearer and disposed between the front waistline region S1 and the rear waistline region S2.

In the present embodiment, the absorbent article 10 may include a front waistline member 20, a rear waistline member 30, and an absorbent body 40. The front waistline member 20 is disposed in the front waistline region S1. The rear waistline member 30 is disposed in the rear waistline region S2. The front waistline member 20 and the rear waistline member 30 may be made of a sheet such as a nonwoven fabric.

The front waistline member 20 may include, for example, a non-skin surface side sheet 14 stacked in two layers and a cover sheet 70 stacked in two layers. In the embodiment illustrated in Fig. 4, the non-skin surface side sheet 14 and the cover sheet 70 are made of the integrally-formed sheet. Specifically, the cover sheet 70 is configured by folding the non-skin surface side sheet 14 at the front edge of the front waistline region S1. Alternatively, the non-skin surface side sheet 14 and the cover sheet 70 may be made of different sheets.

In the embodiment illustrated in Fig. 4, a first elastic member 71 extending in the width direction W is provided near a rear edge 70e of the cover sheet 70. Further, the cover sheet 70 may be bonded to the absorbent body 40 with a bonding member 74, e.g., a hot melt adhesive, on the front side of the first elastic member 71.

A folding line FL which bisects the absorbent article 10 into front and rear portions may pass through the crotch region S3. The folding line FL extends in the width direction W That is, in the state shown in Fig. 1, the absorbent article 10 is folded in half at the folding line FL extending along the width direction W as a base point.

A side engaging portion 60 may be provided at the end portion of the front waistline region S1 in the width direction W and the end portion of the rear waistline region S2 in the width direction W The side engaging portion 60 is defined by a portion where the end portion of the front waistline region S1 in the width direction W and the end portion of the rear waistline region S2 in the width direction W are engaged with each other.

The end portion of the front waistline region S1 in the width direction W and the end portion of the rear waistline region S2 in the width direction W are locked by the side engaging portion 60. In this state, the absorbent article 10, a waistline opening 62 through which the torso is passed and a pair of leg openings 66 into which the legs of the wearer are inserted. A waistline opening edge 64 forming the waistline opening 62 may be defined by the front edge of the front waistline member 20 and the rear edge of the rear waistline member 30. Further, a leg opening edge 68 forming the leg opening 66 may be defined by a side edge 40l of the absorbent body 40 extending in the front-back direction L, a side 20l located behind the front waistline member 20, and a side 30l located in front of the rear waistline member 30.

Fig. 2 illustrates the state in which the side engaging portion 60 is released and the absorbent article 10 is developed. The side engaging portion 60 may extend in the front-back direction L on the front waistline member 20 and the rear waistline member 30. In this case, the boundary between the front waistline region S1 and the crotch region S3 may be defined by the rear edge of the side engaging portion 60 provided on the front waistline member 20. Similarly, the boundary between the rear waistline region S2 and the crotch region S3 may be defined by the front edge of the side engaging portion 60 provided on the rear waistline member 30.

One or more elastic members 32 that are stretchable in the width direction W may be provided in the rear waistline region S2. Alternatively, the elastic members 32 of the rear waistline region S2 may be made of a stretchable sheet that can expand and contract in the width direction W The elastic member 32 may be provided at a position at least partially overlapping an absorbent core 51.

One or more second elastic members 22 that are stretchable in the width direction W may be provided in the front waistline region S1. The second elastic member 22 is made of elastic threads. The second elastic members 22 may be provided on the non-skin surface side of the absorbent body 40 in the front waistline region S1. Further, a stretchable sheet 90 stretchable in the width direction W may be provided in the front waistline region S1. The stretchable sheet 90 may be provided in a region not overlapping the second elastic members 22 in the thickness direction T.

The absorbent body 40 is disposed across the front waistline member 20 and the rear waistline member 30. That is, the absorbent body 40 extends over the front waistline region S1, the rear waistline region S2, and the crotch region S3.

The absorbent body 40 may be formed separately from the front waistline member 20 and the rear waistline member 30. In this case, the absorbent body 40 may be bonded to the front waistline member 20 and the rear waistline member 30, respectively, in the front waistline region S1 and the rear waistline region S2.

The absorbent body 40 at least includes the absorber 50. The absorbent body 40 may also include a skin surface sheet 41 positioned on the skin surface side of the absorber 50 and a non-skin surface sheet 42 positioned on the non-skin surface side of the absorber 50. The skin surface sheet 41 may cover the absorber 50 on the skin surface side of the absorber 50. The non-skin surface sheet 42 may cover the absorber 50 on the non-skin surface side of the absorber 50.

The skin surface sheet 41 may be made of a liquid impermeable sheet, such as a nonwoven fabric or a perforated plastic film. The skin surface sheet 41 may be made of one sheet. Alternatively, the skin surface sheet 41 may be made of a stacked sheet in which a plurality of sheets are stacked.

The non-skin surface sheet 42 may include a liquid impermeable sheet. The liquid impermeable sheet may be made of a liquid impermeable film, such as a plastic film. The non-skin surface sheet42 may be made of one sheet. Alternatively, the non-skin surface sheet42 may be made of a laminated sheet in which a plurality of sheets is laminated. In this case, at least one of the plurality of sheets may be liquid impermeable.

The absorber 50 may include the absorbent core 51 and a core wrap 52 wrapping the absorbent core. The absorbent core 51 may include, for example, ground pulp or a superabsorbent polymer (SAP), or a mixture of them. The core wrap 52 may be made of tissue or tissue paper, for example.

The absorbent core 51 is arranged at least in the crotch region S3. Preferably, the absorbent core 51 may extend from the front waistline region S1 to the rear waistline region S2 in the front-back direction L.

The absorbent core 51 may include a constricted region CA constricted inward in the width direction W at least in the crotch region S3. The constricted region CA may extend in the front-back direction L relative to the folding line FL. The narrowest portion LA of the absorbent core 51 in the width direction W may be provided in the crotch region S3. Further, the narrowest portion of the absorbent core 51 may extend in the front-back direction L. In this case, the folding line FW may pass through the constricted region CAof the absorbent core 51.

The absorbent body 40 may include a side sheet 43 covering both side portions of the skin surface sheet 41 in the width direction W. The side sheet 43 may be made of, for example, a sheet, such as a nonwoven fabric or a perforated plastic film.

The absorbent body 40 may include a pair of leakage preventing cuffs 46. The pair of leakage preventing cuffs 46 extends in the front-back direction L between the front waistline region S 1 and the rear waistline region S2, and is disposed on both sides of the center of the absorbent article 10 in the width direction W The pair of leakage preventing cuffs 46 is defined by a portion (a portion of range B in Fig. 2) that can rise toward the skin of the wearer.

The leakage preventing cuffs 46 may be made of the side sheet 43 and a third elastic member 47 provided on the side sheet 43. The third elastic member 47 is configured to be stretchable in the front-back direction. The third elastic member 47 may include one or more rubber threads. In a specific example, the inner side of the side sheet 43 in the width direction W is folded back to the outside, and the third elastic member 47 is disposed in the folded back portion of the side sheet 43. The inner edge of the side sheet 43 in the width direction W is not bonded to the skin surface sheet 41, and forms a free end that can rise from the skin surface sheet 41.

The pair of leakage preventing cuffs 46 each includes a cuff bonding portion 46j bonded in non-rising manner toward the skin surface side on the rear side of the rear edge of each leakage preventing cuff 46. In the illustrated example, the cuff bonding portion 46j is defined by the bonded portion between the side sheet 43 and the skin surface sheet 41. The side sheet 43 and the skin surface sheet 41 may be bonded by an adhesive such as a hot melt adhesive. Although not illustrated, the pair of leakage preventing cuffs 46 may also include the cuff bonding portion in non-rising manner toward the skin surface side on the front side of the front edge of each leakage preventing cuff 46.

Each leakage preventing cuff 46 includes a rising fulcrum 46a and a rising apex 46b. The rising fulcrum 46a and the rising apex 46b extend in the front-back direction L from the front waistline region S1 to the rear waistline region S2. The rising fulcrum 46a is a non-rising point and corresponds to the root of the leakage preventing cuff 46. In the present embodiment, the rising fulcrum 46a corresponds to an edge line on the inner side in the width direction W of the bonding portion between the side sheet 43 and the skin surface sheet 41. The rising apex 46b corresponds to the apex of the leakage preventing cuff 46 when the leakage preventing cuff 46 rises. That is, the rising apex 46b is the free end of the leakage preventing cuff 46.

The absorbent body 40 may include a pair of gathers 48 in a region outside the pair of leakage preventing cuffs 46 in the width direction W The pair of gathers 48 may extend in the front-back direction L from the front waistline region S1 to the rear waistline region S2.

The pair of gathers 48 may be made of a fourth elastic member 49 attached to a sheet that forms the absorbent body 40. The fourth elastic member 49 may be, for example, one or more rubber threads. Alternatively, the fourth elastic member 49 may be, for example, an elastic sheet having elasticity. The fourth elastic member 49 is bonded to a sheet that forms the absorbent body 40 in a manner stretching from its natural state. Accordingly, the fourth elastic member 49 can form gathers to contract the absorbent body 40 in the front-back direction L.

### (3) Structure of Absorbent Core

Next, the configuration of the absorbent core 51 will be described in detail. Fig. 4 is a plan view of the absorbent core 51 of the absorbent article 10.

The absorbent core 51 may include a high rigidity region HR. The high rigidity region HR is provided behind the folding line FL and in a region including the center in the width direction W. The high rigidity region HR is defined by a region having rigidity higher than the rigidity in a region between the folding line FL and the high rigidity region HR. Preferably, the high rigidity region HR may be formed of an embossed region in which the absorbent core 51 is compressed in the thickness direction.

In addition, the absorbent core 51 may have a pair of grooves 54 formed on both sides with respect to the center of the absorbent article 10 in the width direction W. Here, the "groove" of the absorbent core 51 is a concept including both a recess portion formed in the absorptive material constituting the absorbent core 51 and a region where the basis weight of the absorptive material is zero.

The absorbent core 51 may have a rear slit 56 and a center slit 59 extending forward from the rear side edge of the absorbent core 51 in the rear waistline region S2. In addition, the absorbent core 51 has a pair of side slits 58 located at both outside edges of the absorbent core 51 in the width direction W. Here, the slit may be a region where the absorptive material constituting the absorbent core 51 is substantially zero.

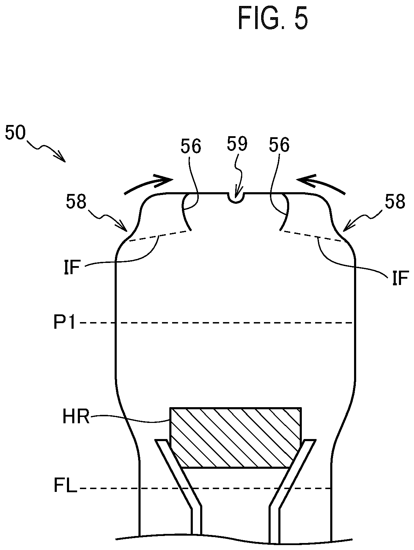

The inner edge part located on the innermost side of the pair of side slits 58 in the width direction W is located on the rear side relative to a line P1 which is in the middle between the rear end edge of the absorbent core 51 and the folding line FL. By the action of the elastic member 32 expanding and contracting in the width direction W, the absorbent core 51 receives a force inward in the width direction W in the rear waistline region S2. Here, the pair of side slits 58 serve as the folding origin of the absorbent core 51, and the absorbent core 51 easily deforms inward in the width direction W from the pair of side slits 58 as the origin in the rear waistline region S2, and is easily bent in a longitudinal direction L from an imaginary line IL connecting together the inner edge parts located at the innermost sides of the pair of side slits 58 as the origin. Due to this, the outer end part in the width direction W of the absorbent core 51 in the rear waistline region S2 moves inward in the width direction and deforms toward the skin side (See also Fig. 5). By this deformation, the part of the absorbent core 51 corresponding to the wearer's buttocks becomes easy to have a cup shape in response to the curvature of the wearer's buttocks.

Furthermore, since the pair of side slits 58 serve as the folding origin of the absorbent core 51, the absorbent core 51 on the rear side relative to the imaginary line IL connecting together the inner edge parts located on the innermost side of the pair of side slits 58 can be suppressed from deforming with the deformation of the absorbent core 51 in the crotch region S3. Accordingly, during wearing, the absorbent core 51 on the rear side relative to the imaginary line IL connecting together the inner edge parts located on the innermost side of the pair of side slits 58 becomes easy to maintain a state of being fitted to the body on the dorsal side of the wearer.

It is preferable that the inner edge part located on the innermost side of the pair of side slits 58 is preferably located on the rear side relative to the narrowest portion LA where the width of the absorbent core 51 is the narrowest in the constricted region CA. The constricted region CA of the absorbent core 51 is a region sandwiched by the legs of the wearer. Accordingly, in the constricted region CA, the rigidity becomes high by crush of the absorbent core 51 in the width direction. Since the inner edge parts of the pair of side slits 58 are provided at positions away from the region rearward, it is possible to further function the action of deforming the absorbent core 51 with the side slits 58 as the origin.

More preferably, the inner edge part located on the innermost side of the pair of side slits 58 is located on the rear side relative to the constricted region CA. In this manner, by arranging the pair of side slits 58 at positions away from the constricted region CA, it is possible to further function the action of deforming the absorbent core 51 with the side slits 58 as the origin.

It is preferable that at least a part, and preferably the entire, of the pair of side slits 58 are provided in a region overlapping the side engaging portion 60 when viewed from the width direction W. The region overlapping the side engaging portion 60 when viewed from the width direction W is a region fitted to the waistline of the wearer. Therefore, the absorbent core 51 is easy to receive a force inward in the width direction W in the region overlapping the side engaging portion 60. Since the pair of side slits 58 are provided in the region overlapping the side engaging portion 60 when viewed from the width direction W, the outside part in the width direction W of the absorbent core 51 in the rear waistline region S2 becomes easier to move inward in the width direction W, and becomes easy to have a cup shape in response to the curvature of the wearer's buttocks.

The maximum length of the pair of side slits 58 in the front-rear direction L is preferably longer than the thickness of the absorbent core 51. More specifically, the maximum length of the pair of side slits 58 is preferably longer than the thickness of the part of the absorbent core 51 adjacent to the side slits. Due to this, when the absorbent core 51 deforms into a cup shape in the rear waistline region S2, the absorbent cores 51 adjacent to each other in the front-rear direction L across the side slit 58 can be suppressed from overlapping each other. This makes it easy for the absorbent core 51 to have a rounded cup shape in accordance with the wearer's body.

The elastic member 32 in the rear waistline region S2 may include a rear-side elastic member on the rear side relative to the pair of side slits 58 and a front-side elastic member on the front side relative to the pair of side slits 58. In this case, the contraction force of the rear-side elastic member is preferably higher than the contraction force of the front-side elastic member. Alternatively, the region between the folding line FL and the pair of side slits 58 may not be provided with an elastic member extending and contracting in the width direction W. That is, the front-side elastic member may not be provided.

Due to this, the absorbent core 51 located on the rear side relative to the pair of side slits 58 receives a force directed inward in the width direction W more strongly. Accordingly, the outside part in the width direction W of the absorbent core 51 in the rear waistline region S2 deforms so as to move further inward in the width direction W, and becomes easy to have a cup shape in response to the curvature of the wearer's buttocks.

As described above, the absorbent core 51 has the rear slit 56 extending forward from the rear end edge of the absorbent core 51. As described above, the absorbent core 51 in the rear waistline region S2 tends to deform inward in the width direction W from the side slit 58 as the origin. Here, if the rear waistline region S2 is provided with the rear slit 56 extending forward from the rear side edge, the absorbent core 51 becomes easier to deform inward in the width direction W

It is preferable that the part located on the innermost side of the side slit 58 in the width direction W is provided on the front side relative to the front side edge of the rear slit 56. In the case where the absorbent core 51 deforms inward in the width direction W in the rear waistline region S2, distortion due to the deformation of the absorbent core 51 can occur up to the front side edge of the rear slit 56. Since the part located on the innermost side of the side slit 58 in the width direction W is provided on the front side relative to the front side edge of the rear slit 56, the side slit can mitigate this distortion. Accordingly, deformation of the absorbent core 51 in the rear waistline region S2 is difficult to be transmitted to the crotch region S3, and only the absorbent core 51 in the rear waistline region S2 is easy to deform into a desired shape.

It is preferable that the part located on the innermost side of the side slit 58 in the width direction W is located near the front side edge of the rear slit 56 relative to the middle line P1 between the rear end edge of the absorbent core 51 and the folding line FL. That is, a distance L1 in the front-rear direction between the part located on the innermost side of the side slit 58 in the width direction W and the front side edge of the rear slit 56 is shorter than a distance L2 in the front-rear direction between the part located on the innermost side of the side slit 58 in the width direction W and the middle line P1. By bringing the part located on the innermost side of the side slit 58 close to the front side edge of the rear slit 56, the absorbent core 51 on the rear side relative to the side slit 58 can be made easier to deform inward in the width direction W by the action of both the side slit 58 and the rear slit 56. This makes it easy for the absorbent core 51 in the rear waistline region S2 to have a cup shape corresponding to the curvature of the wearer's buttocks.

The absorbent core 51 preferably has a pair of rear slits 56 located on both sides across the center in the width direction W. The absorbent core 51 exists between the pair of rear slits 56. Therefore, even in the case where the body fluid runs immediately behind from the center in the width direction of the absorbent core 51, the absorbent core 51 between the pair of rear slits 56 can absorb the body fluid. Due to this, even if the rear slit 56 making it easy to deform the absorbent core 51 exists, it is possible to suppress the leakage of the body fluid from behind.

It is preferable that at least a part, and preferably the entire, of the rear slits 56 is provided on the rear side relative to the part B that can rise of the leakage preventing cuff 46. Since the leakage preventing cuff 46 extending in the front-rear direction L contracts back and forth, if the rear slit 56 of the absorbent core 51 is provided on the front side relative to the part B that can rise of the leakage preventing cuff 46, the absorbent core 51 is becomes difficult to deform inward in the width direction W by a force pulling forward from the leakage preventing cuff 46, thereby making it difficult to have a cup shape. Accordingly, in order to make is easy for the absorbent core 51 have a cup shape in the rear waistline region S2, it is preferable that the rear slit 56 is provided on the rear side relative to the part B that can rise of the leakage preventing cuff 46.

The length of the rear side edge of the rear slit 56 in the width direction W is preferably longer than the length of the front side edge of the rear slit 56 in the width direction W. The rear waistline region S2 tends to contract inward in the width direction W by the elastic member 32 expanding and contracting in the width direction W. Furthermore, the length of the rear side edge of the rear slit 56 in the width direction is longer than the length of the front side edge of the rear slit 56 in the width direction W. Therefore, the absorbent core 51 is made easier to deform inward in the width direction by the action of the elastic member 32 as directed rearward. Due to this, the outer end part of the absorbent core 51 in the width direction deforms toward the skin side while the absorbent core 51 on the outside in the width direction relative to the pair of rear slits 56 moves inward in the width direction (See also Fig. 5). By this deformation, the part of the absorbent core corresponding to the wearer's buttocks becomes easy to have a cup shape in response to the curvature of the wearer's buttocks.

It is preferable that an outer contour 56o outside in the width direction W of the pair of rear slits 56 has a part inclining rearward and outward in the width direction W. More preferably, the entire outer contour 56o outside in the width direction W of the pair of rear slits 56 inclines rearward and outward in the width direction W. Since the outer contour 56o outside the rear slit 56 inclines obliquely, the rear side edge of the outer contour 56o is located outside in the width direction W relative to the front side edge of the outer contour 56o. In this case, the absorbent core 51 in the width direction W relative to the outer contour 56o is located outside in the width direction relative to the position of the front side edge of the outer contour 56o serving as a deformation origin. Accordingly, when the absorbent core 51 in the rear waistline region S2 receives a force inside the width direction W, the absorbent core 51 outside the width direction W relative to the outer contour 56o becomes easier to deform so as to form a cup shape.

It is preferable that an outer contour 56i of the pair of rear slits 56 inside in the width direction W has a part inclining rearward and outward in the width direction W. Since the outer contour 56i of the rear slit 56 obliquely inclines, the outside part of the absorbent core 51 is easier to deform so as to form a cup shape when the absorbent core 51 in the rear waistline region S2 receives a force inside in the width direction W

It is preferable that the rear slit 56 has the maximum length in the width direction W at the rear side edge of the absorbent core 51. Due to this, the absorbent core 51 becomes easy to deform inward in the width direction W as directed rearward. Due to this, in the rear waistline region S2, the absorbent core 51 becomes easier to deform so as to form a cup shape.

It is preferable that the front end edge of the rear slit 56 is pointed inward in the width direction W. Due to this, the absorbent core 51 on the outside relative to the rear slit 56 is easy to deform inward around the front end edge of the rear slit 56. Accordingly, the absorbent core 51 becomes easier to deform so as to form a cup shape in the rear waistline region S2.

The maximum length of the rear slit 56 in the width direction W is preferably equal to or greater than 5 mm. By making the maximum length of the rear slit 56 in the width direction W equal to or greater than 5 mm, it is possible to sufficiently deform the absorbent core 51 in the rear waistline region S2, and it is possible to more easily deform into a cup shape.

The difference between the maximum length of the rear slit 56 in the width direction W and the length of the front end edge of the rear slit 56 in the width direction W is preferably equal to or greater than 10 mm. By sufficiently increasing the difference between the maximum length of the rear slit 56 in the width direction W and the length of the front end edge of the rear slit 56 in the width direction W, the absorbent core 51 becomes easier to deform so as to form a cup shape in the rear waistline region S2.

It is preferable that the length of the absorbent core 51 in the width direction W between the pair of rear slits 56 has a region which becomes longer rearward from the front end edge of the pair of rear slits 56. Since the width of the absorbent core 51 between the pair of rear slits 56 has a part widening rearward, it is possible to suppress the leakage of the body fluid from behind (dorsal side).

It is preferable that the length of the absorbent core 51 in the width direction W between the pair of rear slits 56 becomes maximum on the front side relative to the rear end edge of the absorbent core 51. In the case where the length of the absorbent core 51 in the width direction W between the pair of rear slits 56 becomes maximum at the rear end edge of the absorbent core 51, when the absorbent core 51 deforms inward in the width direction W, the rear end edge of the absorbent core 51 between the rear slits 56 touches the rear end edge of the absorbent core 51 adjacent to the outside the rear slit 56 in the width direction, and the absorbent core 51 may not sufficiently deform so as to close the rear slit 56 during wearing. In the present aspect, since the length of the absorbent core 51 in the width direction between the pair of rear slits 56 becomes maximum on the front side relative to the rear end edge of the absorbent core 51, the absorbent core 51 can sufficiently deform so as to close the rear slit during wearing. The absorbent core 51 on the outside in the width direction relative to the pair of rear slits 56 becomes possible to deform rearward the absorbent core 51 between the pair of rear slits 56, and it becomes easy to form a deformed state in which the absorbent core 51 is pressed against the intergluteal cleft of the wearer. Therefore, it is possible to further suppress leakage of feces from behind.

More preferably, the maximum width of the absorbent core 51 between the pair of rear slits 56 is equal to or greater than 15 mm. By increasing the width of the absorbent core 51 between the pair of rear slits 56, it is possible to further suppress leakage of body fluid from behind (dorsal side).

As mentioned earlier, the absorbent core 51 may have the center slit 59 extending from the rear side edge toward the front side between the pair of rear slits 56. In this case, the length of the center slit 59 in the front-rear direction L is preferably shorter than the length of the pair of rear slits 56 in the front-rear direction L. More preferably, the length of the center slit 59 in the width direction W is shorter than the length of the rear slit 56 in the width direction W. By such the center slit 59, the absorbent core 51 becomes easy to deform inward in the rear waistline region S2. Even in this case, since the center slit 59 in the front-rear direction L is small, it is possible to suppress leakage of body fluid from behind (dorsal side).

It is preferable that at least a part, and preferably the entire, of the pair of rear slits 56 overlap the elastic member 32 in a thickness direction T. It is to be noted that in the case where the elastic member 32 is constituted of an elastic sheet, the entirety of the pair of rear slits 56 can overlap the elastic member 32 in the thickness direction T. Due to this, the contraction by the elastic member 32 is easy to act on a portion of the rear slit 56 of the absorbent core 51, and hence the absorbent core 51 becomes easy to deform further inward in the rear waistline region S2.

More preferably, the contraction force in the width direction W of the rear waistline region S2 on the rear side relative to the rear slit 56 is larger than the contraction force in the width direction W of the rear waistline region S2 on the front side relative to the rear slit 56. For example, the contraction force in the width direction W of the rear waistline region S2 having been cut by a width of 3 cm from the rear slit 56 to the rear side is larger than the contraction force in the width direction W of the rear waistline region S2 having been cut by a width of 3 cm from the rear slit 56 to the front side. In the case where the distance between the rear side edge of the rear slit 56 and the rear side edge of the absorbent article is less than 3 cm, the contraction force in the width direction W of the rear waistline region S2 where the entire region on the rear side has been cut from the rear slit 56 is only required to be larger than the contraction force in the width direction W of the rear waistline region S2 where the region on the front side has been cut from the rear slit 56 with the same width as that of the region on the rear side from the rear slit 56. Due to this, it is possible to make the absorbent core 51 easy to deform strongly in the width direction W in a portion of the rear end edge relative to the front end edge of the rear slit 56. Due to this, the absorbent core 51 becomes easier to deform so as to form a cup shape.

Here, the contraction force in the width direction W of the rear waistline region S2 can be defined as follows. The contraction force can be measured by an electromechanical universal material testing machine (tensile tester: Instron, Model 5564, manufactured by Instron). Specifically, first, a test piece to be measured is prepared. In the case where the absorbent article has a side bonded portion, the side bonded portion is released to develop the absorbent article. Next, in the stretched state in which the absorbent article is extended, a test piece having been cut by the width of 3 cm from the rear slit 56 to the rear side and a test piece having been cut by the width of 3 cm from the rear slit 56 to the front side are acquired. It is to be noted that as described above, in the case where the distance between the rear side edge of the rear slit 56 and the rear side edge of the absorbent article is less than 3 cm, it is only required to acquire a test piece where the entire region on the rear side has been cut from the rear slit 56 and a test piece where the region on the front side has been cut from the rear slit 56 so as to be the same width as the test piece of the region on the rear side from the rear slit 56. Next, both end parts of the test piece of each region having been cut are held by a chuck (holding tool) of the tensile tester. Next, in a state where one of the chucks in the width direction W is fixed, the other chuck is reciprocated by two reciprocations so as to change the distance between the chucks. The moving speed of the chuck at this time is 300 mm/min. It is to be noted that the timing to change the moving direction of the chuck is a point of 95% at the time of maximum stretch. The stress applied on the chuck is measured while the chuck is in motion, and the stress obtained at 60% from the maximum stretch is defined as the "contraction force" of each portion in the front waistline region S1. The maximum stretch refers to a state in which the sheet is stretched up to such an extent that wrinkles are not generated in the sheet by the contraction force of the elastic member.

In the present embodiment, the numerical values relating to the "length" of each part of the absorbent article 10 are measured in the stretched state of the absorbent article 10 stretched until no wrinkles (excluding the folding line FL) are present in the absorbent article 10 when the absorbent article 10 is developed by releasing the side engaging portion 60.

Although the embodiment of the present invention has been described above in detail, it is apparent for persons who have ordinary skill in the art that the embodiment described in this specification does not limit the scope of the present invention. Changes and modifications may apply to the embodiment of the present invention without departing from the scope of the present invention that is defined by the appended claims. The description of the present specification, therefore, has been understood to be illustrative and is in no way intended to limit the scope of the invention.

For example, in the above embodiment, the absorbent body 40 is configured separately from the front waistline member 20 and the rear waistline member 30. Alternatively, the absorbent body 40 may be formed integrally with the front waistline member 20 and the rear waistline member 30.

In the above embodiment, the absorbent article 10 is a so-called underpants type absorbent article having the side engaging portion 60. Alternatively, the absorbent article 10 may be a tape type absorbent article. In this case, the absorbent article 10 does not have the side engaging portion 60, and may have a fastening tape that detachably fixes the front waistline region S1 and the rear waistline region S2 to each other. Preferably, the fastening tape is provided in the rear waistline region S2, and fastens the rear waistline region S2 to the front waistline region S1. In this case, the pair of side slits 58 are preferably provided on the front side relative to the fastening tape. The fastening tape is fastened in a state of being pulled outward in the width direction. When the front side (crotch side) relative to the fastening tape is provided with the pair of side slits 58, the contraction force is not inhibited from acting inward in the width direction at the position of the pair of side slits 58. For this reason, the outside part in the width direction of the absorbent core in the rear waistline region becomes easier to move inward in the width direction, and becomes easy to have a cup shape in response to the curvature of the wearer's buttocks.

### [INDUSTRIAL APPLICABILITY]

According to the above embodiment, it is possible to provide an absorbent article that makes it easier to deform the portion of the absorbent core corresponding to the wearer's buttocks into a cup shape and can suppress leakage of body fluid.

### [REFERENCE SIGNS LIST]

- 10: Absorbent article
- 40: Absorbent body
- 50: Absorber
- 51: Absorbent core
- 54: Groove
- 56: Rear slit
- 58: Side slit
- 59: Center slit
- CA: Constricted region
- S1: Front waistline region
- S2: Rear waistline region
- S3: Crotch region
- FL: Folding line
- L: Front-rear direction
- W: Width direction
- T: Thickness direction

## Claims

1. An absorbent article (10), comprising:
a front-rear direction (L);
a width direction (W) orthogonal to the front-rear direction;
a front waistline region (S1);
a rear waistline region (S2);
a crotch region (S3) arranged between the front waistline region and the rear waistline region;
an absorbent core (51) extending at least from the crotch region to the rear waistline region; and
an elastic member (32) at least partially overlapping the absorbent core (51) in the rear waistline region and expanding and contracting in the width direction, wherein
a folding line (FL) for folding the absorbent article in half extends along the width direction in the crotch region,
the absorbent core (51) has a pair of side slits (58) located at both outside edges of the absorbent core in the width direction, and
an inner edge part located on an innermost side of the pair of side slits (58) in the width direction is located on a rear side relative to a line (P1) which is in the middle between a rear end edge of the absorbent core (51) and the folding line (FL),
wherein the absorbent core (51) has a rear slit (56) extending forward from a rear end edge in the rear waistline region.

2. The absorbent article according to claim 1, wherein
the absorbent core (51) has a constricted region (CA) constricted inward in the width direction at least in the crotch region (S3), and
the inner edge part located on an innermost side of the pair of side slits (58) is located on a rear side relative to a position where a width of the absorbent core (51) is narrowest in the constricted region (CA).

3. The absorbent article according to claim 2, wherein the inner edge part located on an innermost side of the pair of side slits (58) is located on a rear side relative to the constricted region (CA).

4. The absorbent article according to any one of claims 1 to 3, wherein a maximum length of the pair of side slits (58) in the front-rear direction is longer than a thickness of the absorbent core (51).

5. The absorbent article according to any one of claims 1 to 4, wherein
the elastic member (32) includes a rear-side elastic member on a rear side relative to the pair of side slits (58) , and a front-side elastic member on a front side relative to the pair of side slits (58), and
a contraction force of the rear-side elastic member is higher than a contraction force of the front-side elastic member.

6. The absorbent article according to any one of claims 1 to 4, wherein a region between the folding line (FL) and the pair of side slits (58) is not provided with an elastic member extending and contracting in the width direction.

7. The absorbent article according to any one of claims 1 to 6, comprising:
a side engaging portion (60) engaging the front waistline region (S1) and the rear waistline region (S2), wherein
the pair of side slits (58) are provided in a region overlapping the side engaging portion (60) when viewed from the width direction.

8. The absorbent article according to any one of claims 1 to 6, comprising:
a fastening tape provided in the rear waistline region (S2) and fastening the rear waistline region (S2) to the front waistline region (S1), wherein
the pair of side slits (58) are provided on a front side relative to the fastening tape.

9. The absorbent article according to any one of claims 1 to 8, wherein a part located on an innermost side of the side slit (58) in the width direction is provided on a front side relative to a front side edge of the rear slit (56).

10. The absorbent article according to any one of claims 1 to 9, wherein a part located on an innermost side of the side slit (58) in the width direction is located near a front side edge of the rear slit (56) relative to the middle line (P1) between a rear end edge of the absorbent core (51) and the folding line (FL).

11. The absorbent article according to any one of claims 1 to 10, wherein the rear slit (56) is provided on both sides with respect to a center in the width direction.

12. The absorbent article according to claim 11, wherein an outer contour (56ₒ) outside in the width direction of the pair of rear slits (56) has a part inclining rearward and outward in the width direction.

13. The absorbent article according to claim 11 or 12, wherein an outer contour inside (56i) in the width direction of the pair of rear slits (56) has a part inclining rearward and outward in the width direction.

14. The absorbent article according to any one of claims 1 to 13, wherein a front end edge of the rear slit (56) is pointed inward in the width direction.

## Patentansprüche

1. Absorbierender Artikel (10), der Folgendes umfasst:
eine Vorn-Hinten-Richtung (L);
eine Breitenrichtung (W) rechtwinklig zu der Vorn-Hinten-Richtung;
einen vorderen Taillenbereich (S1);
einen hinteren Taillenbereich (S2);
einen Schrittbereich (S3), der zwischen dem vorderen Taillenbereich und dem hinteren Taillenbereich angeordnet ist;
einen Saugkern (51), der sich mindestens von dem Schrittbereich zu dem hinteren Taillenbereich erstreckt; und
ein elastisches Element (32), das zumindest teilweise mit dem Saugkern (51) in dem hinteren Taillenbereich überlappt und sich in der Breitenrichtung dehnt und
zusammenzieht, wobei:
sich eine Faltlinie (FL) zum Falten des absorbierenden Artikels zur Hälfte entlang der Breitenrichtung in dem Schrittbereich erstreckt,
der Saugkern (51) ein Paar von Seitenschlitzen (58) aufweist, die sich an beiden äußeren Rändern des Saugkerns in der Breitenrichtung befinden und
sich ein innerer Randteil, der sich auf einer innersten Seite des Paars von Seitenschlitzen (58) in der Breitenrichtung befindet, auf einer hinteren Seite relativ zu einer Linie (P1) befindet, die in der Mitte zwischen einem hinteren Endrand des Saugkerns (51) und der Faltlinie (FL) vorliegt,
wobei der Saugkern (51) einen hinteren Schlitz (56) aufweist, der sich von einem hinteren Endrand in dem hinteren Taillenbereich nach vorn erstreckt.

2. Absorbierender Artikel nach Anspruch 1, wobei
der Saugkern (51) einen begrenzten Bereich (CA) aufweist, der nach innen in der Breitenrichtung zumindest in dem Schrittbereich (S3) begrenzt ist, und
sich der innere Randteil, der sich auf einer innersten Seite des Paars von Seitenschlitzen (58) befindet, auf einer hinteren Seite relativ zu einer Position befindet, wo eine Breite des Saugkerns (51) in dem begrenzten Bereich (CA) am schmalsten ist.

3. Absorbierender Artikel nach Anspruch 2, wobei sich der innere Randteil, der sich auf einer innersten Seite des Paars von Seitenschlitzen (58) befindet, auf einer hinteren Seite relativ zu dem begrenzten Bereich (CA) befindet.

4. Absorbierender Artikel nach einem der Ansprüche 1 bis 3, wobei eine maximale Länge des Paars von Seitenschlitzen (58) in der Vorn-Hinten-Richtung länger ist als eine Dicke des Saugkerns (51).

5. Absorbierender Artikel nach einem der Ansprüche 1 bis 4, wobei
das elastische Element (32) ein hinterseitiges elastisches Element auf einer hinteren Seite relativ zu dem Paar von Seitenschlitzen (58) und ein vorderseitiges elastisches Element auf einer vorderen Seite relativ zu dem Paar von Seitenschlitzen (58) einschließt und
eine zusammenziehende Kraft des hinterseitigen elastischen Elements größer ist als eine zusammenziehende Kraft des vorderseitigen elastischen Elements.

6. Absorbierender Artikel nach einem der Ansprüche 1 bis 4, wobei ein Bereich zwischen der Faltlinie (FL) und dem Paar von Seitenschlitzen (58) nicht mit einem elastischen Element, das sich in der Breitenrichtung dehnt und zusammenzieht, bereitgestellt ist.

7. Absorbierender Artikel nach einem der Ansprüche 1 bis 6, der Folgendes umfasst:
einen Seiteneingriffsteil (60), der in den vorderen Taillenbereich (S1) und den hinteren Taillenbereich (S2) eingreift, wobei
das Paar von Seitenschlitzen (58) in einem Bereich bereitgestellt ist, der mit dem Seiteneingriffsteil (60) bei Betrachtung von der Breitenrichtung überlappt.

8. Absorbierender Artikel nach einem der Ansprüche 1 bis 6, der Folgendes umfasst: ein Verschlussband, das in dem hinteren Taillenbereich (S2) bereitgestellt ist und den hinteren Taillenbereich (S2) mit dem vorderen Taillenbereich (S1) verschließt, wobei das Paar von Seitenschlitzen (58) auf einer vorderen Seite relativ zu dem Verschlussband bereitgestellt ist.

9. Absorbierender Artikel nach einem der Ansprüche 1 bis 8, wobei ein Teil, der sich auf einer innersten Seite des Seitenschlitzes (58) in der Breitenrichtung befindet, auf einer vorderen Seite relativ zu einem vorderen Seitenrand des hinteren Schlitzes (56) bereitgestellt ist.

10. Absorbierender Artikel nach einem der Ansprüche 1 bis 9, wobei sich ein Teil, der sich auf einer innersten Seite des Seitenschlitzes (58) in der Breitenrichtung befindet, nahe eines vorderen Seitenrands des hinteren Schlitzes (56) relativ zu der Mittellinie (P1) zwischen einem hinteren Endrand des Saugkerns (51) und der Faltlinie (FL) befindet.

11. Absorbierender Artikel nach einem der Ansprüche 1 bis 10, wobei der hintere Schlitz (56) auf beiden Seiten in Bezug auf eine Mitte in der Breitenrichtung bereitgestellt ist.

12. Absorbierender Artikel nach Anspruch 11, wobei eine äußere Kontur (56o) außen in der Breitenrichtung des Paars von hinteren Schlitzen (56) einen Teil aufweist, der sich nach hinten und nach außen in der Breitenrichtung neigt.

13. Absorbierender Artikel nach Anspruch 11 oder 12, wobei eine Innenseite der äußeren Kontur (56i) in der Breitenrichtung des Paars von hinteren Schlitzen (56) einen Teil aufweist, der sich nach hinten und nach außen in der Breitenrichtung neigt.

14. Absorbierender Artikel nach einem der Ansprüche 1 bis 13, wobei ein vorderer Endrand des hinteren Schlitzes (56) nach innen in der Breitenrichtung gerichtet ist.

## Revendications

1. Article absorbant (10), comprenant :
une direction d'avant en arrière (L) ;
une direction de largeur (W) orthogonale à la direction d'avant en arrière ;
une région de ceinture frontale (S1) ;
une région de ceinture dorsale (S2) ;
une région d'entrejambe (S3) arrangée entre la région de ceinture frontale et la région de ceinture dorsale ;
un centre absorbant (51) s'étendant au moins de la région d'entrejambe à la région de ceinture dorsale ; et
un membre élastique (32) chevauchant au moins partiellement le centre absorbant (51) dans la région de ceinture dorsale et s'élargissant et se contractant dans la direction de largeur, dans lequel
une ligne de pliage (FL) pour plier l'article absorbant en deux s'étend le long de la direction de largeur dans la région d'entrejambe,
le centre absorbant (51) a une paire de fentes latérales (58) situées aux deux bords extérieurs du centre absorbant dans la direction de largeur, et
une partie de bord interne située sur un côté le plus intérieur de la paire de fentes latérales (58) dans la direction de largeur est située sur un côté arrière par rapport à une ligne (P1) qui est au milieu entre un bord d'extrémité arrière du centre absorbant (51) et la ligne de pliage (FL),
dans lequel le centre absorbant (51) a une fente arrière (56) s'étendant vers l'avant d'un bord d'extrémité arrière dans la région de ceinture dorsale.

2. Article absorbant selon la revendication 1, dans lequel
le centre absorbant (51) a une région resserrée (CA) resserrée vers l'intérieur dans la direction de largeur au moins dans la région d'entrejambe (S3), et
la partie de bord interne située sur un côté le plus intérieur de la paire de fentes latérales (58) est située sur un côté arrière par rapport à une position à laquelle une largeur du centre absorbant (51) est la plus étroite dans la région resserrée (CA).

3. Article absorbant selon la revendication 2, dans lequel la partie de bord interne située sur un côté le plus intérieur de la paire de fentes latérales (58) est située sur un côté arrière par rapport à la région resserrée (CA).

4. Article absorbant selon l'une quelconque des revendications 1 à 3, dans lequel une longueur maximale de la paire de fentes latérales (58) dans la direction d'avant en arrière est plus longue qu'une épaisseur du centre absorbant (51).

5. Article absorbant selon l'une quelconque des revendications 1 à 4, dans lequel
le membre élastique (32) comprend un membre élastique latéral arrière sur un côté arrière par rapport à la paire de fentes latérales (58), et un membre élastique latéral avant sur un côté avant par rapport à la paire de fentes latérales (58) ; et
une force de contraction du membre élastique latéral arrière est plus élevée qu'une force de contraction du membre élastique latéral avant.

6. Article absorbant selon l'une quelconque des revendications 1 à 4, dans lequel une région entre la ligne de pliage (FL) et la paire de fentes latérales (58) n'est pas pourvue d'un membre élastique s'élargissant et se contractant dans la direction de largeur.

7. Article absorbant selon l'une quelconque des revendications 1 à 6, comprenant :
une partie d'engagement latérale (60) engageant la région de ceinture frontale (S1) et la région de ceinture dorsale (S2), dans lequel
la paire de fentes latérales (58) sont fournies dans une région chevauchant la partie d'engagement latérale (60) lorsque vue de la direction de largeur.

8. Article absorbant selon l'une quelconque des revendications 1 à 6, comprenant :
une bande de fixation fournie dans la région de ceinture dorsale (S2) et attachant la région de ceinture dorsale (S2) à la région de ceinture frontale (S1), dans lequel
la paire de fentes latérales (58) sont fournies sur un côté avant par rapport à la bande de fixation.

9. Article absorbant selon l'une quelconque des revendications 1 à 8, dans lequel une partie située sur un côté le plus intérieur de la fente latérale (58) dans la direction de largeur est fournie sur un côté avant par rapport à un bord latéral avant de la fente arrière (56).

10. Article absorbant selon l'une quelconque des revendications 1 à 9, dans lequel une partie située sur un côté le plus intérieur de la fente latérale (58) dans la direction de largeur est située près d'un bord latéral avant de la fente arrière (56) par rapport à la ligne médiane (P1) entre un bord d'extrémité arrière du centre absorbant (51) et la ligne de pliage (FL).

11. Article absorbant selon l'une quelconque des revendications 1 à 10, dans lequel la fente arrière (56) est fournie des deux côtés par rapport à un centre dans la direction de largeur.

12. Article absorbant selon la revendication 11, dans lequel un contour externe (56ₒ) à l'extérieur dans la direction de largeur de la paire de fentes arrière (56) a une partie s'inclinant vers l'arrière et vers l'extérieur dans la direction de largeur.

13. Article absorbant selon la revendication 11 ou 12, dans lequel un intérieur de contour externe (56ᵢ) dans la direction de largeur de la paire de fentes arrière (56) a une partie s'inclinant vers l'arrière et vers l'extérieur dans la direction de largeur.

14. Article absorbant selon l'une quelconque des revendications 1 à 13, dans lequel un bord d'extrémité avant de la fente arrière (56) est orienté vers l'intérieur dans la direction de largeur.
